(19) Europäisches Patentamt
European Patent Office
Office européen des brevets

(11) EP 2 314 302 B1

(12) **EUROPEAN PATENT SPECIFICATION**

(45) Date of publication and mention of the grant of the patent:
**02.05.2018 Bulletin 2018/18**

(21) Application number: **10005579.7**

(22) Date of filing: **28.05.2010**

(51) Int Cl.:
*A61K 36/16* (2006.01)        *A61L 9/01* (2006.01)
*A62B 23/00* (2006.01)        *B01D 46/00* (2006.01)
*F24F 3/16* (2006.01)

(54) **Composition for preventing infection of new influenza A (H1N1) virus comprising ginkgo extract, air filter comprising the same, and air cleaning device comprising the filter**

Zusammensetzung zur Prävention einer Infektion mit dem neuen Influenza-A-(H1N1-) Virus, die Ginkgoextrakt umfasst, Luftfilter, der diese Zusammensetzung umfasst, und Luftreinigungsvorrichtung, die den Filter umfasst

Composition pour empêcher l'infection du nouveau virus de la grippe A (H1N1) comportant un extrait de ginkgo, filtre à air la comportant et dispositif de nettoyage d'air comportant le filtre

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **14.10.2009 US 251672 P**

(43) Date of publication of application:
**27.04.2011 Bulletin 2011/17**

(73) Proprietor: **Woongjin Coway Co., Ltd.**
**Gongju-si**
**Chungcheongnam-do**
**314-895 (KR)**

(72) Inventors:
• **Kim, Hyoung Joon**
**San 4-1**
**Nakseongdae-dong**
**Gwanak-gu**
**Seoul 151-919 (KR)**
• **Park, Chan Jung**
**San 4-1**
**Nakseongdae-dong**
**Gwanak-gu**
**Seoul 151-919 (KR)**

(74) Representative: **Kador & Partner**
**Corneliusstraße 15**
**80469 München (DE)**

(56) References cited:
**EP-A1- 1 813 338         EP-A1- 1 946 815**
**WO-A2-2010/047550**

• **DATABASE WPI Week 200914 Thomson Scientific, London, GB; AN 2009-E98205 XP002609224, & KR 2008 088 900 A (WOONG JIN COWAY CO LTD) 6 October 2008 (2008-10-06)**
• **SCHWERDTFEGER S M ET AL: "Wirkung von Pflanzenextrakten auf die Neuraminidase-Aktivität = Effects of plant extracts on neuraminidase activity", ZEITSCHRIFT FUER PHYTOTHERAPIE, HIPPOKRATES VERLAG IN MVS MEDIZINVERLAGE, DE, vol. 29, no. 2, 1 January 2008 (2008-01-01), pages 65-70, XP009133398, ISSN: 0722-348X, DOI: DOI:10.1055/S-2008-1077269**
• **MIKI ET AL: "Anti-influenza virus activity of biflavonoids", BIOORGANIC & MEDICINAL CHEMISTRY LETTERS, PERGAMON, ELSEVIER SCIENCE, GB, vol. 17, no. 3, 19 January 2007 (2007-01-19), pages 772-775, XP005835936, ISSN: 0960-894X, DOI: DOI:10.1016/J.BMCL.2006.10.075**

## Description

BACKGROUND OF THE INVENTION

1. Field of the Invention

The present invention relates to a composition for the prophylaxis of the new influenza A (H1N1) virus infection, comprising a ginkgo extract, an air filter comprising the same, and an air cleaner comprising the air filter.

2. Description of the Related Art

The 2009 flu pandemic which began in March, 2009 represented a global outbreak of a new strain of H1N1 influenza virus. It is often referred to as the "swine flu". However, because the influenza A virus subtype H1N1 cannot be spread by eating pork or pork products, it has been renamed as a new flu or as the new influenza A (H1N1) in Korea. The World Health Organization (WHO) declared the outbreak to be a pandemic on June 11, 2009.

According to governmental statistical data, more than ten thousand cases of the new flu were generated with eight serious cases hospitalized and 14 patients dying from chronic or acute complications, as of Sep. 16, 2009.

Among antiviral drugs currently available for the treatment of influenza are oseltamivir (trade name: Tamiflu), zanamivir (trade name: Relenza), peramivir and amantadine, with the predominant application of Tamiflu to the treatment of influenza A virus subtype H1N1. Tamiflu, a drug with a worldwide monopoly, was developed to treat avian influenza (AI). By blocking the activity of the viral neuraminidase enzyme, oseltamivir prevents new viral particles from being released by infected cells. An effective efficacy can be obtained when it is taken within 48 hours of the onset of symptoms. The main efficacy of Tamiflu is known to lie in the relief of symptoms, the warding off of secondary complications such as bronchitis or pneumonia, and the decrease of the latent period. Tamiflu has been used to treat and prevent influenzavirus A and influenzavirus B infections in tens of millions of people since 1999. Zanamivir, sold under the trade name Relenza, is a neuraminidase inhibitor used in the treatment and prophylaxis of Influenzavirus A and Influenzavirus B.

Side effects associated with oseltamivir therapy include nausea and vomiting. Zanamivir shows high antiviral effects, but poor bioavailability, with fast release from the kidney.

Most of the anti-influenza agents developed thus far have side effects. Thus, there is a need for the development of an anti-influenza composition that is effective for the treatment and prophylaxis of influenza virus infection.

Commonly, influenza is spread via an airborne route, that is, when someone inhales the aerosols produced by the coughing, sneezing or spitting of an infected person. Influenza can also be spread by direct transmission, e.g., by way of excretions, spits, snivel, and blood of infected persons. However, the spread of influenza results most commonly from droplet infection such as aerosol inhalation. Influenza viruses may be inactivated by sun light, disinfectants, surfactants, e.g., soap, which are, however, not effective for the removal of airborne viruses.

The capture and inactivation of airborne viruses may lead to the effective prevention of influenza infection.

An air filter is an air-permeable member which can removes solid particulates from the air. Air filters are used in applications where air quality is important, such as in air cleaners, air conditioners, vacuum cleaners, humidifiers, dehumidifiers, etc., notably in building ventilation systems and in engines.

Designed to remove contaminants from the air, an air cleaner comprises a plurality of filters composed typically of a pretreatment filter for removing large size particles; a deodorizing filter for removing odor, volatile organic chemicals, formaldehyde, etc.; an HEPA filter for removing airborne particulates in micro-size diameter; and a median filter, arranged in front of the HEPA filter, for protecting the HEPA filter.

More filters may result in higher air-purifying performance, but increase resistance against overall air circulation. Accordingly, 3 to 5 filters are typically employed in an air cleaner.

Recently, studies have focused on functional filters which can selectively remove harmful materials in an elaborate manner or substitute beneficial materials for harmful materials. For example, filters for removing microparticulates with high efficiency or for purifying airborne particulates which cause sick house syndrome have been developed.

Ginkgo biloba is the only extant species within the order Ginkgoales.

Ginkgos are very large trees, normally reaching a height of over 30 m and a diameter of 2.5 m. The tree has a pyramidal figure and is sparsely branched. The bark of long-lived ginkgos is gray in color and furrowed deeply, and has a texture like that of cork. The wood is faint colored and almost valueless economically because it is light and weak. The leaves are usually 8 cm long with a width double that of the length. The leaves are fan-shaped with veins radiating out into the leaf blade, sometimes bifurcating from the central V-shaped split. Two veins enter the leaf blade at the base and fork repeatedly in two. The leaves are tinged with grayish or yellowish green in Summer, turn to golden yellow in Autumn, and fall off in the late Autumn. In herbal medicine, the dried bare seeds are known to clean the lungs and the stomach and to be useful in the treatment of cough and phlegm. Ginkgo flavone glycoside, found in ginkgo leaves, is used to improve blood circulation in the body.

For instance, KR 2008 0088 900 A relates to an air purifying filter comprising a gingko extract and an air cleaner comprising the air purifying filter for removing allergens and influenza virus in air.

Further, WO 2010/047550 A2 is concerned with an air purifier having a dehumidification function for the removal of moisture from inflow air and the purification of inflow air. Optionally, the air purifier may include a functional filter removing an influenza virus, an avian influenza virus, and the like by the use of contained non-toxic natural materials such as sumac and gingko extract. Furthermore, EP 1 813 338 A1 refers to an air filter comprising natural extract components from gingkoes being contained in a breathable support and an air processing device comprising the air filter, able to inactivate an allergen trapped on the filter. Further, an antiviral activity against influenza virus A (H1N1) is disclosed.

EP 1 946 815 A1 discloses an anti-allergen filter containing an anti-allergen agent such as gingko leaf extract on a first filter and an air cleaning system using the anti-allergen filter.

The scientific paper of Schwerdtfeger et al., "Wirkung von Pflanzenextrakten auf die Neuraminidase-Aktivität", Zeitschrift für Phytotherapie (2008), 29, 65-70, describes that methanolic gingko extract can act as a strong neuraminidase-inhibitor *in vitro.*

Moreover, the scientific paper of Miki et al., "Anti-influenza virus activity of biflavonoids", Bioorganic & Medicinal Chemistry Letters (2007), 17, 772-775, relates to the inhibitory activity of biflavonoids isolated from Gingko extracts against influenza virus sialidase. In particular, the inhibition of the proliferation of influenza virus strains A/PR/8/34 (H1N1), A/Guizhou/54/89 (H3N2) and B/Ibaraki/2/85 by biflavonoids such as ginkgetin is disclosed.

Leading to the present invention, intensive and thorough research into a composition acting against new influenza A H1N1, conducted by the present inventors, resulted in the finding that a ginkgo extract has an inhibitory activity against new influenza A (H1N1) and that a composition comprising the ginkgo extract as an active ingredient is useful in the prophylaxis of influenza A (H1N1) virus infection and a filter coated with the extract can effectively remove airborne influenza A (H1N1) viruses.

## SUMMARY OF THE INVENTION

It is therefore an object of the present invention to provide a composition for the prevention of new influenza A (H1N1) virus infection, comprising a ginkgo extract.

It is another object of the present invention to provide an air filter comprising the composition.

It is a further object of the present invention to provide an air cleaner comprising the filter.

In accordance with an aspect thereof, the present invention provides a composition for use in the prevention of influenza A (H1N1) virus infection, comprising a ginkgo extract as an active ingredient, wherein the influenza A (H1N1) is A/California/04/09.

In a preferred embodiment, the composition may be applied to quasi-drugs.

In accordance with another aspect thereof, the present invention provides the use of an air filter for preventing influenza A (H1N1) virus infection comprising the composition, wherein the influenza A (H1N1) is A/California/04/09.

It accordance with a further aspect thereof, the present invention provides the use of an air cleaner for preventing influenza A (H1N1) virus infection, comprising the air filter, wherein the influenza A (H1N1) is A/California/04/09.

## DESCRIPTION OF THE PREFERRED EMBODIMENTS

The present invention addresses a composition for use in the prophylaxis of new influenza A (H1N1) virus infection, comprising a ginkgo extract as an active ingredient, wherein the influenza A (H1N1) is A/California/04/09.

As used herein, the term "prevention" or "prophylaxis" is intended to include all actions suppressing new influenza A (H1N1) virus infection or deterring the outbreak of influenza.

Influenza A (H1N1) virus is a subtype of influenza A virus and is the most common cause of influenza (flu) in humans. Some strains of H1N1 are endemic to humans and cause a small fraction of all influenza-like illness. Other strains of H1N1 are endemic to pigs (swine influenza) and to birds (avian influenza). Examples of the influenza A include A/PR/8(H1N1), A/WSN/33(H1N1), A/Bervig-Mission/1/18(rvH1N1), and A/Singapore/6/86(H1N1).

Among the new influenza A (H1N1) virus, which is responsible for the 2009 flu pandemic, are A/California/O4/09 and A/California/7/2009.

When infected with a new influenza A (H1N1) virus, patients suffer from fever, cough, sore throat, bronchitis, pneumonia, etc.

The extract may be obtained from a Ginkgo biloba material which may be purchased commercially, taken from nature, or cultivated artificially.

Examples of the ginkgo material useful in the present invention include leaves, seeds, bark and roots, with a preference for leaves.

So long as it is applied to extraction from a ginkgo material, any method, e.g., from a simple extraction method to a method of extracting lipid-soluble ingredients may be employed in the present invention. For the convenience of extraction, the material may be pulverized first, followed by extraction with a solvent, filtration and concentration.

Examples of the extraction solvents useful in the present invention include water, ethanol, methanol, butanol, n-hexane, n-heptane, DMSO, and a combination thereof. An artisan skilled in the art may choose a suitable extraction method depending on various factors including the amount of materials, extraction type, time, temperature, efficiency, solvents, etc. Preference is given to an extraction time of from 10 min to 1 hr and an extraction temperature of from 40 to 100°C.

In addition, the extract thus obtained may be filtered using a well-known method, such as vacuum filtration, and then concentrated by evaporation. Such a series of extraction, filtration and concentration is well known in the art. Those skilled in the art may select suitable techniques for the processes to afford a ginkgo extract.

In accordance with another embodiment of the present invention, the composition for use in the prophylaxis of A/California/O4/09 virus infection may be applied in the preparation of quasi-drugs.

With the aim of preventing infection with A/California/O4/09, the composition of the present invention may be used as an additive in a quasi-drug. In this regard, the composition may be used alone or in combination with another quasi-drug or ingredient in a typical manner. The amount of the composition in the quasi-drug may be determined depending on the purpose thereof.

Examples of the quasi-drug to which the composition of the present invention may be applied include a hand-wash, a mouthwash, a disinfectant, a shower foam, a water tissue, a detergent soap and a mask.

In accordance with a further embodiment thereof, the present invention addresses the use of an air filter comprising the composition for the prophylaxis of new influenza A (H1N1) virus infection, wherein the influenza A (H1N1) is A/California/04/09.

The term "air filter", as used herein, refers to a filter which functions to remove airborne microorganisms and dust and which prevents secondary contamination attributable to a filter. The air filter of the present invention may be thus applied to automobile cabins, household electric appliances, air conditioning systems, gas masks, air cleaners, and clean rooms, with a preference for air cleaners.

In addition to the composition of the present invention, the filter according to the present invention may comprise a conventional antibacterial agent, a deodorant (e.g., a flavonoid, phytoncide, pyroligneous liquor, a plant extract, cyclodextrin, metal ion, or titanium dioxide), a dust collecting agent, etc. These agents may be applied individually or in combination, with no particular limitations imparted to the order of coating.

In accordance with a further embodiment thereof, the present invention addresses the use of an air cleaner equipped with the air filter for preventing influenza A (H1N1) virus infection, wherein the influenza A (H1N1) is A/California/04/09.

As mentioned above, an air cleaner comprises a plurality of filters. The air filter comprising the composition for the prophylaxis of new influenza A (H1N1) virus infection can be employed as a functional filter in the air cleaner.

No limitations are imparted to the type of the air cleaner to which the air filter of the present invention is applied. It may be applied to air cleaners for home, offices, and automobile cabins. Of course, the air cleaner may comprise known typical constitutional factors. Preferably, the air cleaner of the present invention comprises an air filter placed between an air intake and an air exhaust.

A better understanding of the present invention may be obtained through the following examples which are set forth to illustrate, the present invention.

**PREPARATION EXAMPLE 1: Preparation of Ginkgo Extract**

For extraction efficiency, leaves, taken from ginkgo trees, were dried and pulverized into powder. To 1 kg of the powder was added 5 liters of water, followed by heating in a water bath for 12 hrs. The extract thus obtained was filtered through a filter paper and concentrated.

**PREPARATION EXAMPLE 2: Manufacture of an Air Filter Coated with the Ginkgo Extract**

The ginkgo extract prepared in Preparation Example 1 was diluted in water and sprayed onto a polypropylene filter base which was then dried at 140°C for 4 min.

**EXPERIMENTAL EXAMPLE 1: Assay for inhibitory activity of the Ginkgo Extract against Influenza A (H1N1) Virus**

The ginkgo extract obtained in Preparation Example 1 was assayed for inhibitory activity against influenza virus as follows.

As influenza virus strains to be tested, influenza A subtype H1N1 viruses A/PR/8 (H1N1) and A/WSN (H1N1) as well as the WHO standard strain influenza A (H1N1) (A/California/O4/09), which was responsible for the declaration of the 2009 pandemic were used. The ginkgo extract obtained in Preparation Example 1 was used as a sample.

The MDCK (Mardine Darbine Canine Kidney) cell line was inoculated at a density of 1.5 x $10^6$ cells/mL into 6-well plates which were then incubated at 37°C for 24 hrs in a 5% $CO_2$ atmosphere. Separately, the ginkgo extract was diluted to 100 mg/ml (1x) in an injection solution. The dilution was subjected to serial 10-fold dilutions. The specimens thus obtained were added in an amount of 90 $\mu$L per well to 96-well plates. 10 uL of an influenza virus sample was added to each well, incubated for 10 min, and 10-fold diluted with PBS. The MDCK cell line which had grown to confluence on the plates was infected with 1 mL of the dilution and incubated for 1 hr. For an "infection + non-administration" control, the MDCK cell line was infected with influenza virus which had not been treated with the ginkgo extract.

Thereafter, the medium was removed, and a mixture of 1:1 2x agarose : 2x MEM containing 10 $\mu$g/ml trypsin was added in an amount of 2 mL per well. The MDCK cells were incubated at 37°C for 2 days, fixed with 1 mL of 4% paraformaldehyde, and washed with water to remove agarose. On the next day, the cells were stained with crystal violet to count plaques and express the titer as plaque forming unit (pfu)/mL. The results are summarized in Tables 1 to 3, below.

[Equation 1]

$$\text{Inhibitory Activity(\%)} = (1 - \text{Ginkgo Extract-Treated Group/Control}) \times 100$$

TABLE 1

| Titer of New Influenza A (H1N1) (A/California/O4/09) after Treatment with Sample | | |
| --- | --- | --- |
| Sample | Virus Titer (pfu/ml) | Inhibitory Activity(%) |
| Control | $1.9 \times 10^6$ | |
| Ginkgo Extract | $1.0 \times 10^2$ | 99.99 |

TABLE 2

| Titer of Influenza A/WSN/33 (H1N1) after Treatment with Sample | | |
| --- | --- | --- |
| Sample | Virus Titer (pfu/ml) | Inhibitory Activity(%) |
| Control | $3.4 \times 10^8$ | - |
| Ginkgo Extract | $7.0 \times 10^3$ | 99.99 |

TABLE 3

| Titer of Influenza A/PR/8 (H1N1) after Treatment with Sample | | |
| --- | --- | --- |
| Sample | Virus Titer (pfu/ml) | Inhibitory Activity (%) |
| Control | $3.9 \times 10^9$ | - |
| Ginkgo Extract | $6.0 \times 10^2$ | 99.99 |

As is apparent from the data of Tables 1 to 3, the group treated with the ginkgo extract was significantly reduced in virus count compared to the control, indicating that the ginkgo extract of the present invention has excellent inhibitory activity against influenza virus.

**EXPERIMENTAL EXAMPLE 2: Assay for inhibitory activity of the Air Filter against Influenza Virus**

The air filter coated with the ginkgo extract, obtained in Preparation Example 2, was assayed for inhibitory activity against influenza A (H1N1) virus as follows.

As influenza virus strains to be tested, influenza A subtype H1N1 viruses A/PR/8 (H1N1) and A/WSN (H1N1) as well as the WHO standard strain influenza A (H1N1) (A/California/O4/09), which was responsible for the declaration of the 2009 pandemic were used. The air filter manufactured in Preparation Example 2 was used as a sample. For control, an air filter which was not treated with the extract was used.

After being cut into a size of 2x2 cm, the filter was coated with a predetermined amount of the virus solution and incubated for 10 min so as to absorb the virus thereinto. A medium (1 mL) was loaded onto the filter which was then shaken for 10 min to wash off the virus. The filtrate was diluted to prepare specimens. 90 μL of each of the specimens was added, together with 10 μL of influenza virus, to each well of 96-well plates, followed by incubation for 10 min and 10-fold dilution with PBS. The MDCK cell line grown to confluence on the plates was infected with 1 mL of the dilution and incubated for 1 hrs. For an "infection + non-administration" control, the MDCK cell line was infected with influenza virus which had not been treated with the ginkgo extract.

Thereafter, the medium was removed, and a mixture of 1:1 2x agarose : 2x MEM containing 10 μg/ml trypsin was added in an amount of 2 mL per well. The MDCK cells were incubated at 37°C for 2 days, fixed with 1 mL of 4% paraformaldehyde, and washed with water to remove agarose. On the next day, the cells were stained with crystal violet to count plaques and express the titer as plaque forming unit (pfu)/mL.

The results are summarized in Tables 4 to 6, below.

TABLE 4

| Titer of New Influenza A (H1N1) (A/California/O4/09) after Treatment with Sample | | |
|---|---|---|
| Sample | Virus Titer (pfu/ml) | Inhibitory Activity(%) |
| Control | $1.9 \times 10^6$ | - |
| Ginkgo Extract-Treated Air Filter | Not-detected | 99.99 |

TABLE 5

| Titer of Influenza A/WSN/33 (H1N1) after Treatment with Sample | | |
|---|---|---|
| Sample | Virus Titer (pfu/ml) | Inhibitory Activity(%) |
| Control | $3.4 \times 10^8$ | - |
| Ginkgo Extract-Treated Air Filter | Not-detected | 99.99 |

TABLE 6

| Titer of Influenza A/PR/8 (H1N1) after Treatment with Sample | | |
|---|---|---|
| Sample | Virus Titer (pfu/ml) | Inhibitory Activity(%) |
| Control | $3.9 \times 10^9$ | - |
| Ginkgo Extract-Treated Air Filter | Not-detected | 99.99 |

The data of Tables 4 to 6 demonstrate that the air filter treated with the ginkgo extract of the present invention has excellent inhibitory activity against influenza virus.

Having high inhibitory activity against the new influenza A (H1N1) virus, as described hitherto, the composition comprising a ginkgo extract in accordance with the present invention can be applied to the prevention of new influenza A (H1N1) virus infection. Hence, a filter coated with the composition can remove influenza A (H1N1) virus from the air so that it can be employed in an air cleaner for the prophylaxis of new influenza A (H1N1) virus infection.

**Claims**

1.  A composition for use in preventing influenza A (H1N1) virus infection, comprising a ginkgo extract as an active ingredient, wherein the influenza A (H1N1) is A/California/04/09.

2.  The composition for use according to claim 1, wherein the ginkgo extract is obtained by using a solvent selected from among water, ethanol, methanol, butanol, n-hexane, n-heptane, DMSO and combinations thereof.

3. The composition for use according to claim 1, wherein the ginkgo extract is obtained from leaves, seeds, bark or roots of Ginkgo biloba.

4. The composition for use according to claim 1, wherein the influenza A (H1N1) virus causes fever, cough, sore throat, bronchitis, or pneumonia.

5. The composition for use according to claim 1, wherein the composition is used in preparing a quasi-drug, wherein the quasi-drug is selected from a group consisting of a hand-wash, a mouthwash, a disinfectant, a shower foam, a water tissue, a detergent soap, and a mask.

6. Use of an air filter for preventing influenza A (H1N1) virus infection, comprising the composition of claim 1, wherein the influenza A (H1N1) is A/California/04/09.

7. Use of an air cleaner for preventing influenza A (H1N1) virus infection, comprising the air filter of claim 6, wherein the influenza A (H1N1) is A/California/04/09.


**Patentansprüche**

1. Zusammensetzung zur Anwendung bei der Vorbeugung einer Influenza A (H1N1) Virusinfektion, umfassend einen Ginkgo-Extrakt als einen Wirkstoff, wobei der Influenza A (H1N1) Virus A/California/04/09 ist.

2. Zusammensetzung zur Anwendung gemäß Anspruch 1, wobei der Ginkgo-Extrakt durch Verwendung eines Lösungsmittels, ausgewählt aus Wasser, Ethanol, Methanol, Butanol, n-Hexan, n-Heptan, DMSO und Kombinationen davon, erhalten wird.

3. Zusammensetzung zur Anwendung gemäß Anspruch 1, wobei der Ginkgo-Extrakt aus Blättern, Samen, Rinde oder Wurzeln des Ginkgo biloba erhalten wird.

4. Zusammensetzung zur Anwendung gemäß Anspruch 1, wobei der Influenza A (H1N1) Virus Fieber, Husten, Halsschmerzen, Bronchitis oder Lungenentzündung verursacht.

5. Zusammensetzung zur Anwendung gemäß Anspruch 1, wobei die Zusammensetzung zum Herstellen eines Quasi-Arzneimittels verwendet wird, wobei das Quasi-Arzneimittel ausgewählt wird aus der Gruppe bestehend aus einer Handwaschmittel, einem Mundwasser, einem Desinfektionsmittel, einem Duschschaum, einem Wassergewebe, einer Waschmittelseife und einer Maske.

6. Verwendung eines Luftfilters zur Vorbeugung einer Influenza A (H1N1) Virusinfektion, umfassend die Zusammensetzung nach Anspruch 1, wobei der Influenza A (H1N1) Virus A/California/04/09 ist.

7. Verwendung eines Luftreinigers zur Vorbeugung einer Influenza A (H1N1) Virusinfektion, umfassend den Luftfilter nach Anspruch 6, wobei der Influenza A (H1N1) Virus A/California/04/09 ist.


**Revendications**

1. Composition destinée à une utilisation visant à empêcher une infection par le virus de la grippe A (H1N1), qui comprend un extrait de ginkgo en tant que substance active, dans laquelle la grippe A (H1N1) est A / California / 04 / 09.

2. Composition destinée à une utilisation selon la revendication 1, dans laquelle l'extrait de ginkgo est obtenu en utilisant un solvant sélectionné dans le groupe constitué par l'eau, l'éthanol, le méthanol, le butanol, le n - hexane, le n - heptane, le DMSO, et des associations de ceux-ci.

3. Composition destinée à une utilisation selon la revendication 1, dans laquelle l'extrait de ginkgo est obtenu à partir de feuilles, de graines, d'écorce ou de racines de Ginkgo biloba.

4. Composition destinée à une utilisation selon la revendication 1, dans laquelle le virus de la grippe A (H1N1) provoque une fièvre, une toux, une angine, une bronchite, ou une pneumonie.

5. Composition destinée à une utilisation selon la revendication 1, dans laquelle la composition est utilisée dans la préparation d'un quasi-médicament, et dans laquelle le quasi-médicament est sélectionné dans le groupe constitué par un produit pour se laver les mains, un bain de bouche, un désinfectant, une mousse de douche, un tissu imprégné d'eau, un savon détersif et un masque.

6. Utilisation d'un filtre à air visant à empêcher une infection par le virus de la grippe A (H1N1), qui comprend la composition selon la revendication 1, dans laquelle la grippe A (H1N1) est A / California / 04 / 09.

7. Utilisation d'un purificateur d'air visant à empêcher une infection par le virus de la grippe A (H1N1), qui comprend le filtre à air selon la revendication 6, dans laquelle la grippe A (H1N1) est A / California / 04 / 09.

## REFERENCES CITED IN THE DESCRIPTION

### Patent documents cited in the description

- KR 20080088900 A
- WO 2010047550 A2
- EP 1813338 A1
- EP 1946815 A1

### Non-patent literature cited in the description

- **SCHWERDTFEGER et al.** Wirkung von Pflanzenextrakten auf die Neuraminidase-Aktivität. *Zeitschrift für Phytotherapie,* 2008, vol. 29, 65-70
- **MIKI et al.** Anti-influenza virus activity of biflavonoids. *Bioorganic & Medicinal Chemistry Letters,* 2007, vol. 17, 772-775